# EUROPEAN PATENT APPLICATION

(11) **EP 3 012 270 A1**
(43) Date of publication of application: **27.04.2016**
(21) Application number: 14190167.8
(22) Date of filing: 23.10.2014
(51) Int. Cl.: C07K 16/28, A61K 39/395, G01N 33/53

(54) **Products for modulating microbiota composition for improving the efficacy of a cancer treatment with an immune checkpoint blocker**

(71) Applicant: Institut Gustave Roussy, 94800 Villejuif (FR)
(72) Inventor: Zitvogel, Laurence, 75006 Paris (FR); Vetizou, Marie, 92120 Montrouge (FR)
(74) Representative: Santarelli

(57) **Abstract**

The present invention relates to the role of the microbiota in the efficacy of cancer treatments with a drug blocking an immune checkpoint, and provides methods and probiotics to improve the efficacy of such a treatment in patients in need thereof. More particularly, the invention pertains to the use of vancomycin or penicillin to modulate the gut microbiota to potentiate the anticancer effects of anti-CTLA4 molecules. *B. fragilis* can also be used as a probiotic to that aim.

## Description

### FIELD OF THE INVENTION

The present invention relates to the field of anticancer treatment. In particular, the present invention concerns the role of the microbiota in the efficacy of cancer treatments with a drug blocking an immune checkpoint, and provides methods and probiotics to improve the efficacy of such a treatment in patients in need thereof.

### BACKGROUND AND PRIOR ART

Oncogenesis and cancer progression result from a complex interplay between cell-autonomous (epi)genetic instability and the microenvironment. Microbial communities inhabiting our intestine and other portals of entry thus far are poorly appreciated environmental factors potentially impacting on carcinogenesis. Pioneering studies performed in germ-free mice, animals exposed to specific bacteria in specialized facilities (gnotobiotic mice) or in antibiotic-treated mice, revealed an unsuspected role of commensals and pathobionts in accelerating tumorigenesis. Contrasting with these findings, other observations support a beneficial role of some bacteria against cancer. Thus, prolonged treatment of mice expressing transgenic Her2/Neu with a combination of metronidazole and ciprofloxacine actually tripled breast cancer occurrence (Rossini et al, 2006). In humans, several epidemiologic studies revealed a dose-dependent association between antibiotic use and breast cancer risk (Blaser, 2011; Velicer et al, 2004).

Adding some complexity, the clinical management of cancer patients compromises the delicate symbiosis between the gut microbiota and the host. Mucositis, a major oncological problem caused by anticancer chemotherapeutic agents, is worsened by neutropenia and antibiotics (Stringer et al, 2009; van Vliet et al, 2010). The crucial role of gut microbiota in eliciting innate and adaptive immune responses beneficial for the host in the context of effective therapies against cancer was recently reported (Viaud et al, 2014a; Viaud et al, 2014b; Viaud et al, 2013). By compromising intestinal integrity, chemotherapeutic agents enhance gut permeability and favor the selective translocation of Gram⁺ bacteria (*L. johnsonii* + *E. hirae)* into secondary lymphoid organs. There, anti-commensal pathogenic TH17 (pTh17) T cell responses are primed, facilitating the intratumoral accumulation of tumor-specific TH1 T cells that is associated with tumor regression after chemotherapy with cyclophosphamide (CTX) (Viaud et al, 2014a; Viaud et al, 2014b; Viaud et al, 2013). To demonstrate a causal relationship between gut microbiota and systemic pTh17 responses induced by CTX, animals that had been previously sterilized by means of antibiotics were treated with a cocktail of Gram⁺ bacteria (*L. johnsonii* + *E. hirae*), and it was found that this cocktail (but not *L. reuteri* or *L. plantarum*) could induce pTh17 in the spleen of CTX (but not vehicle) -treated animals (Viaud et al, 2013) and restored the CTX- anti-cancer effects lost in antibiotic-treated mice (unpublished data). Importantly, the redox equilibrium of myeloid cells contained in the tumor microenvironment is also influenced by the intestinal microflora, contributing to tumor responses (Iida et al, 2013). Hence, the anticancer efficacy of alkylating agents and platinum salts is compromised in germ-free (GF) mice, as well as in mice treated with antibiotics. These findings represent a paradigm shift in the understanding of the mode of action of conventional chemotherapeutics.

The inventors have now demonstrated that the first-in-class monoclonal antibody targeting an immune checkpoint (anti-CTLA4 mAb, Ipilimumab/YERVOY®) also mediates T cell-dependent antitumor effects *via* an effect on the intestinal microbiota.

Ipilimumab is a fully human monoclonal antibody (mAb) directed against CTLA4, a key negative regulator of T cell activation (Peggs et al, 2006). CTLA4, which is present in intracytoplasmic vesicles of resting T cells, is upregulated in activated T cells and translocates to the plasma membrane to maintain self-tolerance and prevent autoimmunity (Tivol et al, 1995). Ipilimumab is the first FDA- and EMA-approved therapeutic (since 2011) that improves the overall survival of patients with metastatic melanoma (MM) in randomized Phase III trials (Hodi et al, 2010; Robert et al, 2011). After more than 7 year-follow up, it appears that Ipilimumab can achieve about 20% durable (often complete) disease control in MM and other malignancies (Page et al, 2013). However, blockade of CTLA4 by Ipilimumab often results in immune-related adverse events (irAEs) at sites that are exposed to commensal flora, namely the gut and the skin (Beck et al, 2006; Berman et al, 2010; Weber et al, 2009). These irAEs can be of grade III-IV in 20% cases, life threatening (in <5% cases), resistant to steroids and anti-TNFa Ab, thereby causing premature interruption of the therapy. The enterocolitis associated with Ipilimumab has features similar to both graft-versus-host disease and inflammatory bowel disease, the acute phase being characterized by neutrophilic infiltrates while the chronic phase involves granulomata and lymphocytes. Although incriminated in the first place, neither low regulatory T cell (Treg) numbers nor specific genetic traits constitute predictive biomarkers for Ipilimumab-induced enterocolitis. Patients develop antibodies to enteric flora (Berman et al, 2010), suggesting that intestinal commensals or bacterial antigens may contribute to colitis. Despite these deleterious colitogenic effects, investigators currently combine blockade of two immune checkpoints i.e., CTLA4 and PD1 receptors, which further increases the response rate, as well as the incidence and severity of irAEs (Hamid et al, 2013).

Ipilimumab generates micro-abscesses in colons of diseased patients. The formation of abscesses is a pathological response to distinct bacterial pathogens, in particular the anaerobic bacterium *Bacteroides fragilis* (Tzianabos et al, 1993). The ability of *B. fragilis* to cause these infections is tightly linked to the presence of a unique capsular polysaccharide (polysaccharide A, PSA) on this organism. *Bacteroides* zwitterionic polysaccharides (ZPS), which contain alternating positive and negative charges in each repeating unit, are taken up by antigen-presenting cells, processed by the major histocompatibility class II pathway, and presented to T cells in the context of MHC class II molecules (Stingele et al, 2004). After ZPS presentation, CD4⁺ T cells can recognize and respond specifically to these carbohydrates. T cells can be activated *in vitro* by abscess-inducing ZPS in the presence of CD28-B7 co-stimulation and then promote abscess formation when adoptively transferred to the peritoneal cavity of rodents. A blocking CTLA4 Ig prevented abscesses following challenge with *Bacteroides fragilis* or a combination of *Enterococcus faecium* and *Bacteroides distasonis* (Tzianabos et al, 2000a). However, in the context of gut homeostasis, *B. fragilis* induces regulatory T cells (Treg) to secrete the potent anti-inflammatory cytokine IL-10, limiting inflammation in the gut and at distant sites (Mazmanian et al, 2008; Surana & Kasper, 2012). In the absence of antigen presenting cells, PSA elicits IL-10 secretion by CD4⁺ T cells in a TLR2-dependent manner (Round et al, 2011). In a mouse model of colitis, Dasgupta et al. (2014) showed that PSA required both innate and adaptive immune mechanisms to restore gut tolerance. Plasmacytoid dendritic cells (pDC), but not other conventional DC, sense *B. fragilis* PSA through TLR2, upregulate ICOSL and CD86 and activate IL-10-producing Treg in a CD86- and ICOS-dependent manner, promoting anti-inflammatory effects and avoiding colitis (Dasgupta et al, 2014).

*B. fragilis* requires PSA to occupy a mucosal niche in the colon. Hence, *B. fragilis* deltaPSA failed to colonize the colonic crypts and induced significant Th17 responses in the lamina propria. Depletion of Treg and neutralization of IL-17A decreased and markedly increased niche-specific mucosal colonization of *B.fragilis* respectively (Round et al, 2011). There is a species-specific saturable colonization of the colonic crypts by *B. fragilis* via a unique class of polysaccharide utilization loci (PUL) that are highly conserved among intestinal *Bacteroides.* This genetic locus has been named *ccf* for « Commensal Colonization Factors », determines the composition of the bacterial glycocalix and hence *Bacteroides spp.* bio distribution and functions (Huang et al, 2011; Koropatkin et al, 2009; Lee et al, 2013; Martens et al, 2008).

There is therefore a compelling need for the development of improved treatments for cancer which favor a constructive interaction, if not a synergy, between treatments comprising at least a drug blocking an immune checkpoint and immunity. Uncoupling efficacy from gut toxicity also represents an unmet medical need challenging the future development of immune checkpoint blockers as antineoplastic agents.

### SUMMARY OF THE INVENTION

The present invention relates to a combination of a drug blocking an immune checkpoint and of an antibiotic selected from the group consisting of vancomycin and penicillin, for use in the treatment of cancer.

The invention also pertains to the use of vancomycin or penicillin, for modulating the gut microbiota of a patient to potentiate the anticancer effects of a drug blocking an immune checkpoint administered to said patient.

Another object of the present invention is *an in vitro* method of identifying a patient who cannot be a good responder to a drug blocking CTLA4, comprising determining the functionality of the toll-like receptor 4 (TLR 4) in said patient, wherein if said patient lacks a functional TLR 4, the patient is identified as not being a good responder to a drug blocking CTLA4.

A probiotic bacterial composition comprising bacteria selected from the group consisting of *Bactero ides fragilis, Bacteroides thetaiotaomicron* and mixtures thereof, for use in combination with a drug blocking an immune checkpoint for inducing immunostimulation in a cancer patient, is also part of the present invention.

The present invention also relates to the use of a dendritic cell (DC) presenting capsular polysaccharides A (PSA) or PSA and adjuvants or zwitterionic polysaccharide (ZPS) repeated motifs from *B. fragilis* or lysine-aspartic acid (KD) peptides with >15 repetitive units, for use in adoptive cell transfer (DC), in combination with an antineoplastic treatment comprising a drug blocking an immune checkpoint, for treating cancer.

The present invention also relates to the use of a polyclonal T cell line or bulk autologous T cells *ex vivo* expanded with dendritic cells (DC) presenting capsular polysaccharides A (PSA) or PSA and adjuvants or zwitterionic polysaccharide (ZPS) repeated motifs from *B. fragilis* or lysine-aspartic acid (KD) peptides with >15 repetitive units, for use in adoptive cell transfer of T lymphocytes, in combination with an antineoplastic treatment comprising a drug blocking an immune checkpoint, for treating cancer.

The present invention also provides an anticancer vaccine composition comprising PSA and/or KD and/or ZPS, as well as its use in combination with a drug blocking an immune checkpoint, for inducing immunostimulation in a cancer patient.

### LEGENDS TO THE FIGURES

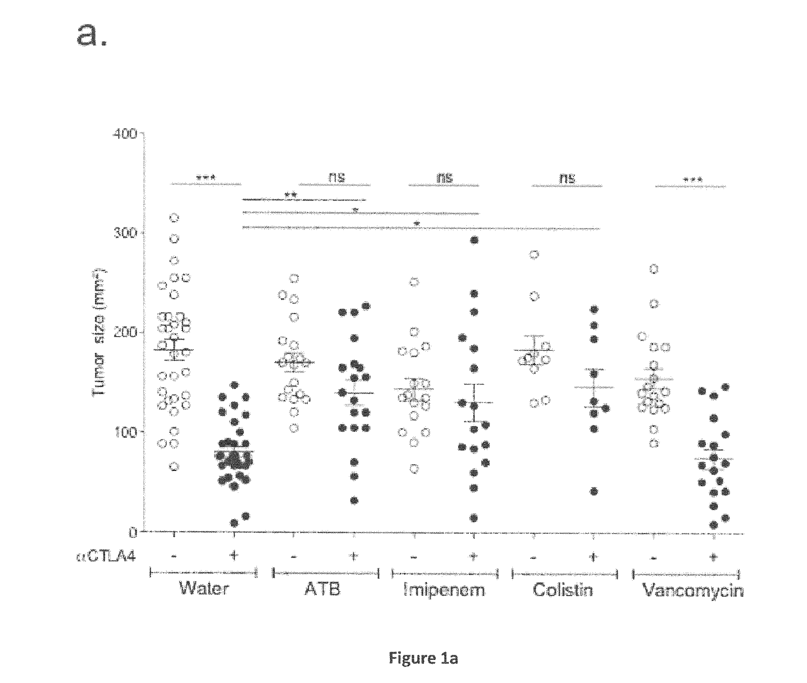

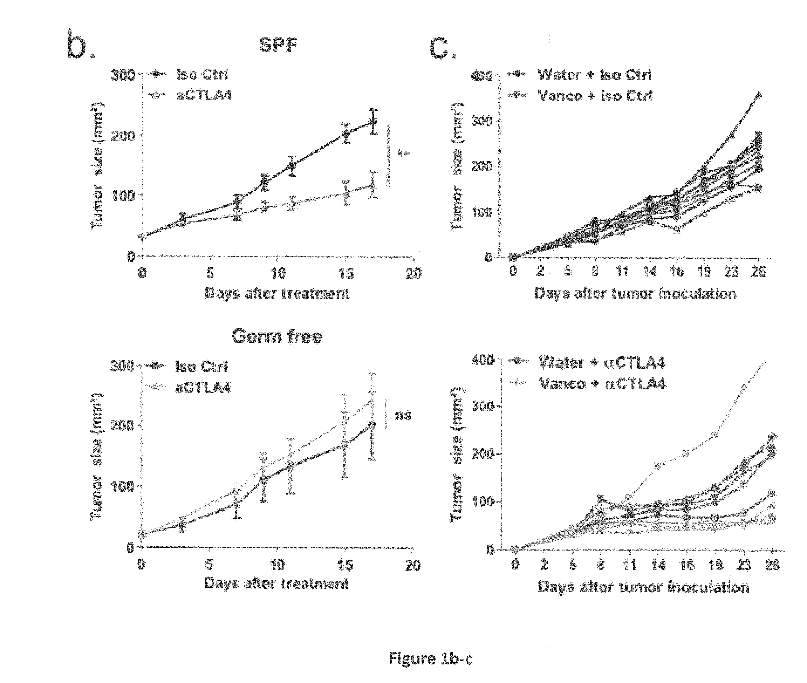

**Figure 1****: The antitumor effects mediated by anti-CTLA4 Ab markedly depend on the gut microbiota.** Prior to tumor inoculation, naive C57BL/6 mice maintained in SFP conditions (a, b) or germ -free (b) were pre-treated with various antibiotic regimens (vs PBS) (SPF only), combination of neomycine, colistine, streptomycine (ATB), imipenem, colistin (a) or vancomycine (c). MCA205 sarcomas were established s.c. at day 0. At day 8, 11, 14, mice received 3 i.p. injections of 100ug anti-CTLA4 Ab (clone 9D9) and tumor growth kinetics were monitored. The graph represents all tumor sizes per individual mouse at sacrifice (a) or overtime (b, c) for 10-15 animals/group. Similar results were obtained in CT26 and MC38 tumor models. Student's t test or Anova:* p<0.05, **p<0 .01, ***p<0.001, ns: not significant.

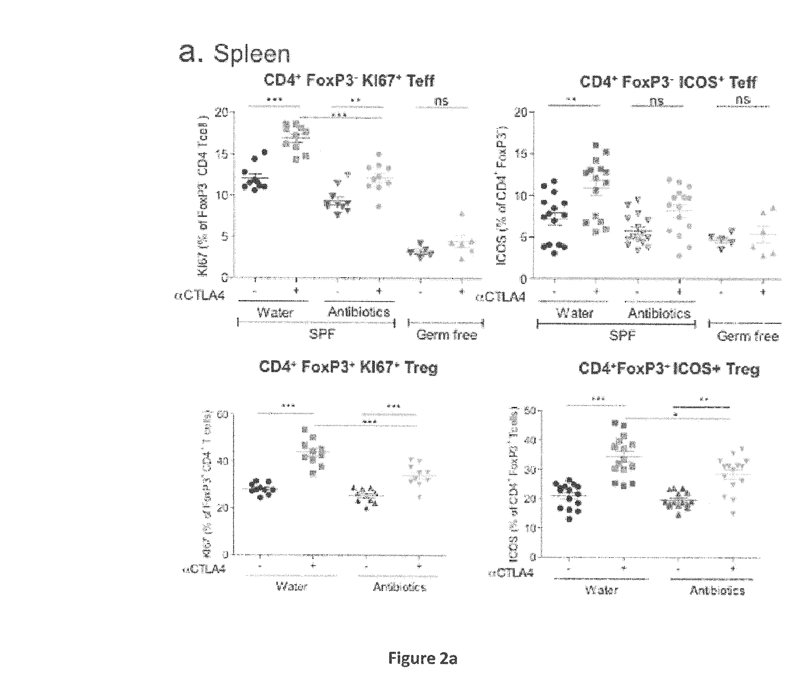

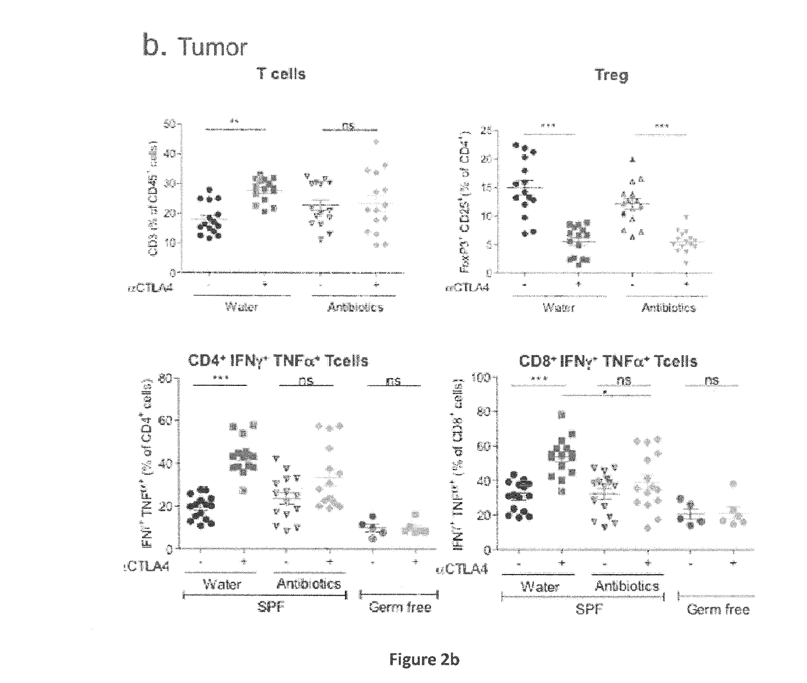

**Figure 2****: Gut microbiota-dependent systemic and tumoral immune effects mediated by CTLA4 blockade.** Similar protocol as in Fig. 1. Spleens (a) and tumors (b) were collected at day 21 to analyze the percentages and functions of conventional and regulatory T cells by flow cytometry (intracellular staining for cytokine detection after PMA-ionomicine stimulation). Graphs represent means+SEM of percentages of positive cells for 5-6 animals/group. Each experiment has been performed twice yielding similar results. SPF and GF mice were sacrificed at day 15 and 21 respectively for these analyses. Student's t test or Anova:* p<0.05, **p<0 .01, ***p<0.001, ns: not significant.

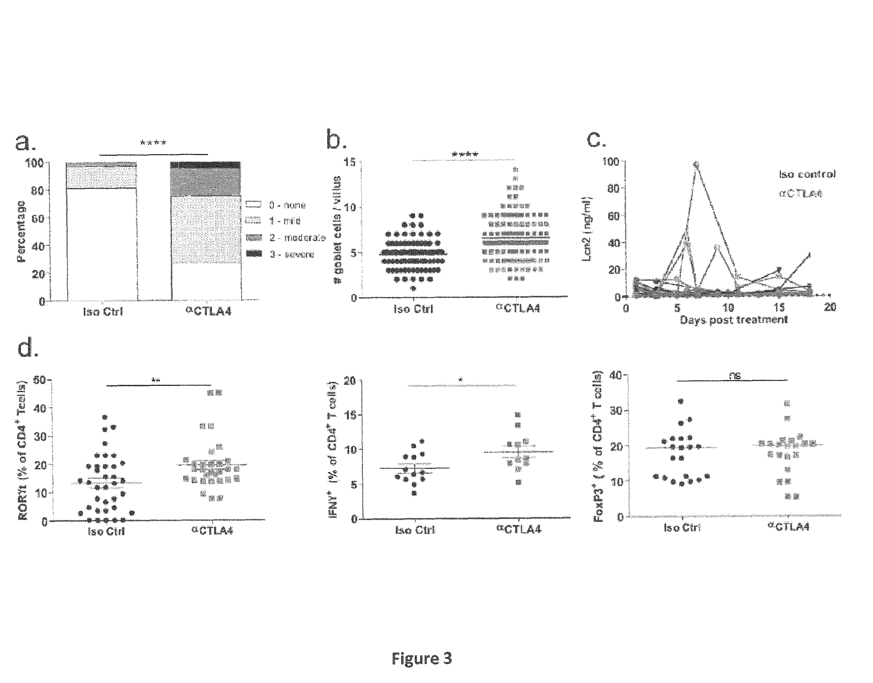

**Figure 3****. Gut toxicity mediated by CTLA4 blockade.** Similar protocol as in Fig. 1. Small and large intestines were collected at day 21 to analyze gross abnormalities by IHC with H&E staining (a), to enumerate goblet cells/villus in the ileum (b), to monitor the concentrations of the antimicrobial peptide Lipocalin-2 in ELISA in the feces (c), to analyze by flow cytometry Treg cells and IFNg-producing or RORgt⁺ CD4⁺ T cells (intracellular staining after PMA-ionomicine stimulation) (d). Graphs represent results of 10 colons/group (a), 5 mice and 50 villi/mouse (b), 5 mouse stools overtime (c)/group. Data in (d) represent individual mice concatanated from three experiments. Each experiment has been performed at least twice yielding similar results. Student's t test or Anova:* p<0.05, ***p<0.001, ns: not significant.

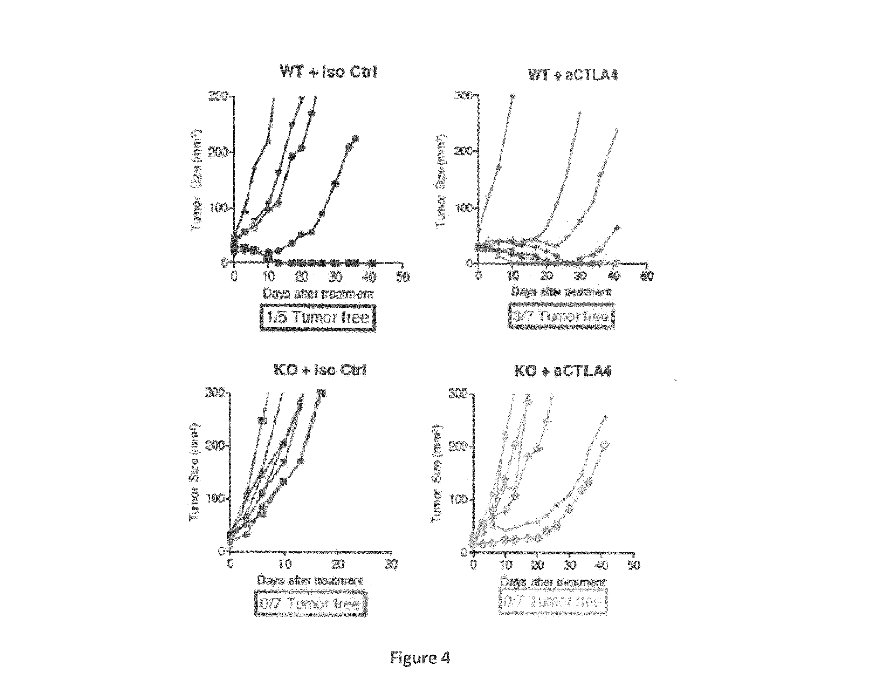

**Figure 4****: Efficacy of CTLA4 blockade in tlr4^{-/-} mice**
   BALB/c mice of a WT or Tlr4^{-/-} background were inoculated with CT26 and treated three times with anti-CTLA4 Ab as detailed above. Tumor growth kinetics were compared in the two mouse species and tumor free mice were recorded. In Tlr4 KO mice, anti-CTLA4 Ab lost part of their tumoricidal activity.

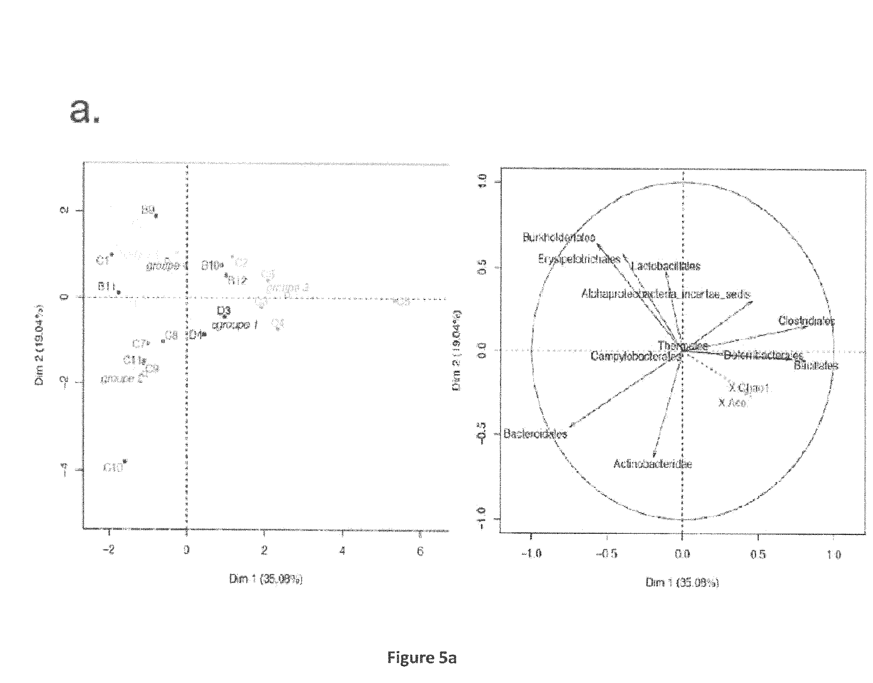

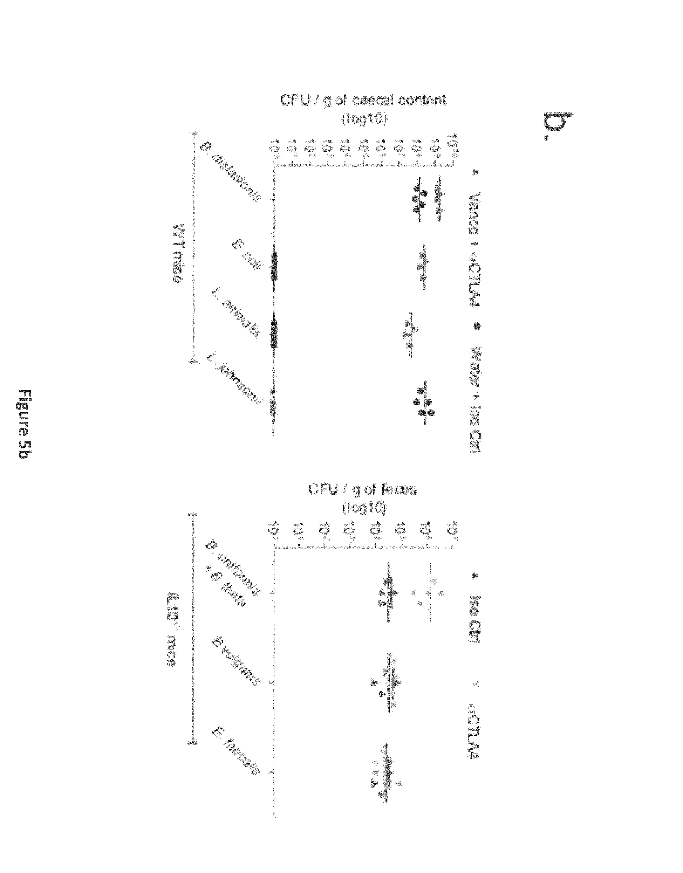

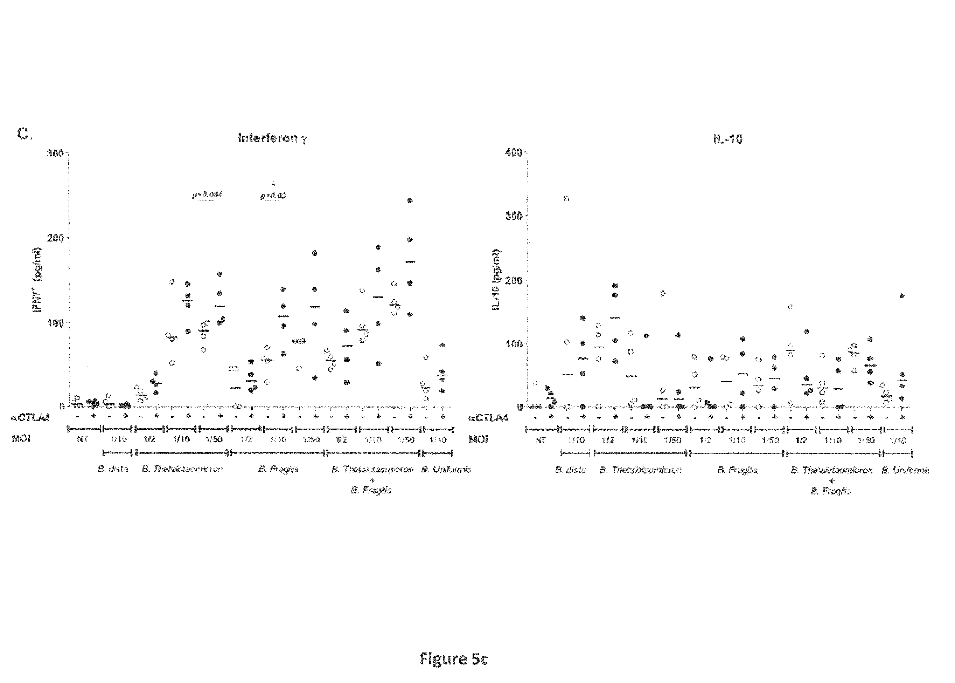

**Figure 5****: CTLA4 blockade induced a dominance of** *Bacteroidales* **over *Clostridiales* in feces of tumor bearers.** The therapeutic regimen is detailed in Fig. 1. a. Pyrosequencing of the 16S rRNA of feces collected after 1 and 2 injections of isotype control Ab (green and dark blue) versus anti-CTLA4 Ab (red and cyan blue) allowed to determine significant variations between the two groups at 1 (green versus red) and 2 injections (dark and cyan blues). PCA are shown for 5 mice/group where individual mouse results are shown with the representative families of bacteria. b. Plating stool from anti-CTLA4 Ab-treated mice in conditions of vancomycine or IL-10 deficiency on agar selective for anaerobes revealed a dramatic enrichment in *Bacteroidales* and to a lesser extent of *E. Coli.* c. Memory T cell responses to various *Bacteroidales species* were tested in splenocytes from mice treated with anti-CTLA4 or isotype control Ab in coculture with bone marrow-derived DC loaded with various *Bacteroidales spp.* for 48 hours and ELISA was performed on supernatants to monitor IFNg and IL-10 release. Anova test: * p<0.05, **p<0.01, ***p<0.001, ns: not significant.

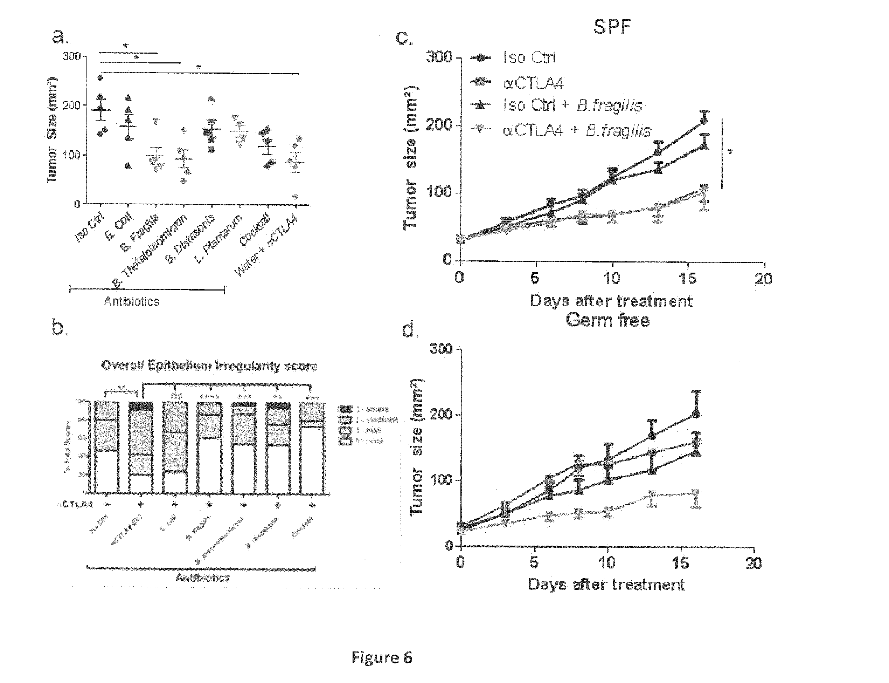

**Figure 6****: *B.fragilis* or *B. thethaiotaomicron* but not *E. Coli* compensated for the loss of antitumor efficacy mediated by anti-CTLA4 Ab in ATB-treated and GF mice.** a. The therapeutic regimen is detailed in Fig. 1. The 14 day long-broad spectrum antibiotic therapy was stopped before the mono-association by oral feeding with different bacterial species (indicated in the graph) and the day before the first injection of anti-CTLA4 Ab. At day 10 post-3rd injection, tumors were measured and reported on this graph. Each dot represents one mouse tumor size. Each group contained 5 mice. Cocktail: all 5 bacteria). Grey dots (right hand side of the graph) represent mice that never received ATB and benefited from anti-CTLA4 Ab. b. Colons were examined at sacrifice as detailed in Fig. 3a. This experiment has been repeated twice, yielding similar conclusions. c-d. Monoassociation of SPF (c) or GF (d) mice with *B*.*fragilis* and tumor growth kinetics during anti-CTLA4 Ab administration. Anova test:* p<0.05, **p<0 .01, ***p<0.001, ns: not significant.

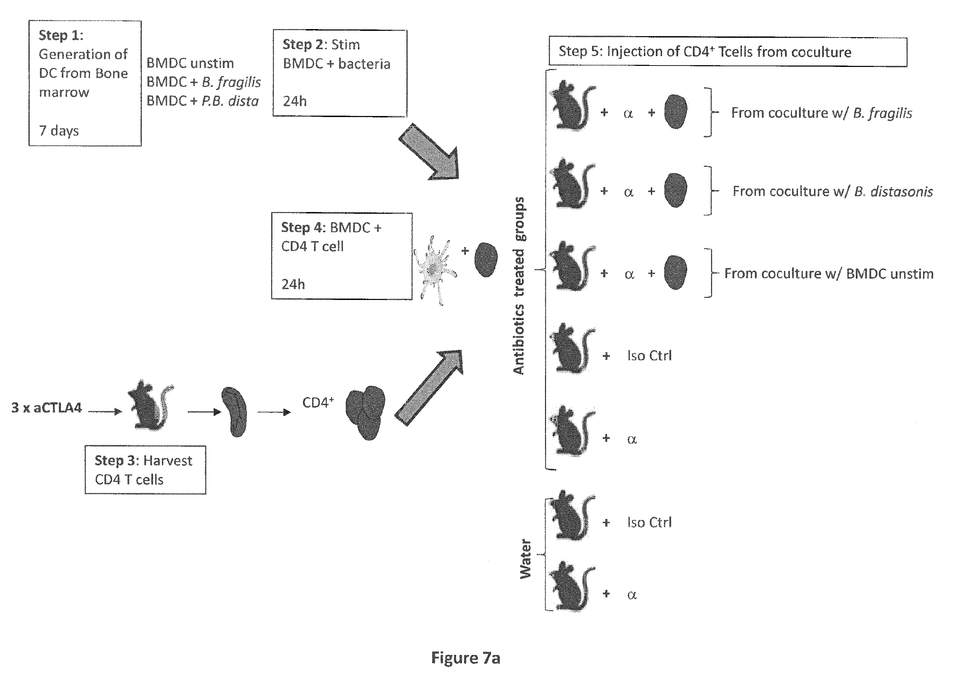

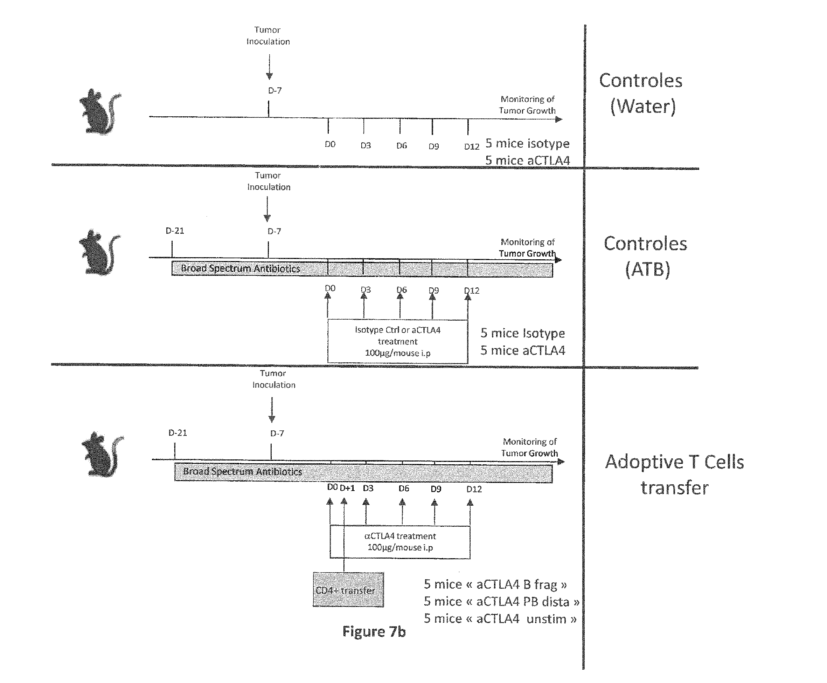

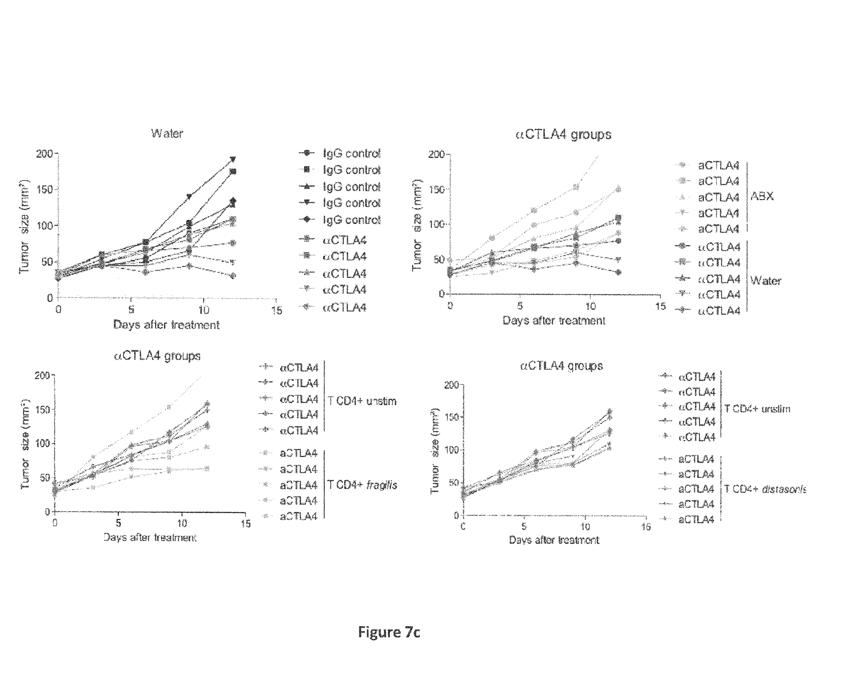

**Figure 7****: Adoptive CD4+ T cell transfer.** MCA205-OVA tumors established in wild-type mice pretreated for 3 weeks with broad spectrum antibiotics were injected with anti-CTLA4. At day 1 after anti-CTLA4 treatment, one million of ex vivo primed CD4⁺ T cells were injected intravenously. T cells were primed ex vivo with bone marrow derived dendritic cells (BM-DC) exposed to Bacteroides *fragilis* or Bacteroides *distasonis* at a MOI 1:10. Only CD4⁺ T cells primed with *Bacteroides,* mainly with *Bacteroides fragilis* mediated anti-CTLA4 anti-tumor efficacy in antibiotics context. Association of anti-CTLA4 and CD4⁺ T cells primed with BM-DC without *Bacteroides* are unable to reduce tumor growth.

### DETAILED DESCRIPTION OF THE PREFERRED EMBODIMENTS

In the present text, the following general definitions are used:

### Gut microbiota

The "gut microbiota" (formerly called gut flora or microflora) designates the population of microorganisms living in the intestine of any organism belonging to the animal kingdom (human, animal, insect, *etc*.). While each individual has a unique microbiota composition (60 to 80 bacterial species are shared by more than 50% of a sampled population on a total of 400-500 different bacterial species/individual), it always fulfils similar main physiological functions and has a direct impact on the individual's health:
- it contributes to the digestion of certain foods that the stomach and small intestine are not able to digest (mainly non-digestible fibers);
- it contributes to the production of some vitamins (B and K);
- it protects against aggressions from other microorganisms, maintaining the integrity of the intestinal mucosa;
- it plays an important role in the development of a proper immune system;
- a healthy, diverse and balanced gut microbiota is key to ensuring proper intestinal functioning.

Taking into account the major role gut microbiota plays in the normal functioning of the body and the different functions it accomplishes, it is nowadays considered as an "organ". However, it is an "acquired" organ, as babies are born sterile; that is, intestine colonisation starts right after birth and evolves afterwards.

The development of gut microbiota starts at birth. Sterile inside the uterus, the newborn's digestive tract is quickly colonized by microorganisms from the mother (vaginal, skin, breast, *etc*.), the environment in which the delivery takes place, the air, *etc.* From the third day, the composition of the intestinal microbiota is directly dependent on how the infant is fed: breastfed babies' gut microbiota, for example, is mainly dominated by *Bifidobacteria,* compared to babies nourished with infant formulas.

The composition of the gut microbiota evolves throughout the entire life, from birth to old age, and is the result of different environmental influences. Gut microbiota's balance can be affected during the ageing process and, consequently, the elderly have substantially different microbiota than younger adults.

While the general composition of the dominant intestinal microbiota is similar in most healthy people (4 main phyla, *i. e., Firmicutes, Bacteroidetes, Actinobacteria* and *Proteobacteria*), composition at a species level is highly personalised and largely determined by the individuals' genetic, environment and diet. The composition of gut microbiota may become accustomed to dietary components, either temporarily or permanently. Japanese people, for example, can digest seaweeds (part of their daily diet) thanks to specific enzymes that their microbiota has acquired from marine bacteria.

### Dysbiosis

Although it can adapt to change and has a high resilience capacity, a loss of balance in gut microbiota composition may arise in some specific situations. This is called "dysbiosis", a disequilibrium between potentially "detrimental" and known "beneficial" bacteria in the gut or any deviation to what is considered a "healthy" microbiota in terms of main bacterial groups composition and diversity. Dysbiosis may be linked to health problems such as functional bowel disorders, inflammatory bowel diseases, allergies, obesity and diabetes. It can also be the consequence of a treatment, such as a cytotoxic treatment or an antibiotic treatment.

A specific dysbiosis can be highlighted depending on the pathogenic condition. For instance, patients with Crohn's disease, a chronic inflammatory bowel disease, present a microbiota with reduced percentages and diversity of bacteria belonging to the Firmicutes phylum, and mostly from the *Clostridium leptum* (cluster IV) group (Manichanh et al., 2006; Sokol et al., 2006).Generally, decreased percentages of bacteria from the Lachnospiraceae family can be observed. Moreover mucosa- associated microbiota of these patients is depleted in bacteria from the *Bifidobacterium* and *Lactobacillus* genera toward increased levels of potentially pathogenic bacteria such as specific strains of *Escherichia coli* with adherent and invasive phenotypes (AIEC) (Darfeuille-Michaud et al., 2004; Joossens et al.).

To the contrary, patients with obesity and metabolic disorders have higher proportions of bacteria belonging to the Firmicutes phylum and lower levels of *Escherichia coli* in their feces (Ley et al., 2005; Turnbaugh et al., 2009). An increased in proportions of *E. coli* in these patients has been associated with weight loss following bariatric surgery and lower levels of serum leptin (Furet et al.).

In patients with colorectal cancer (CRC), however, gut microbial dysbiosis relates to enrichment in bacterial species from the *Bacteroides* genus and decrease of *Faecalibacterium* and *Roseburia* genera belonging species (Sobhani et al., ; Wu et al.). Specifically, *Fusobacterium* and *Campylobacter genera* were found to be consistently increased in both feces and mucosa of CRC patients.

In the context of cancer, "beneficial or "favorable" bacteria are essentially *Lactobacillus* and *Bifidobacterium,* and "detrimental" or "unfavorable" bacteria are essentially the species *Parabacteroides distasonis* and *Faecalibacterium prausnitzii,* the genera *Gemmiger, Alistipes* and *Clostridium Cluster IV. (Clostridium leptum* group).

### Antineoplastic treatments

"Antineoplastic treatments" herein designate any treatment for cancer except surgery. They include chemotherapy, hormonal and biological therapies, and radiotherapy.

### Biological therapies

Anti cancer "biological therapies" involve the use of living organisms, substances derived from living organisms, or laboratory-produced versions of such substances to treat cancer, by targeting either the cancer cells directly, or by stimulating the body's immune system to act against cancer cells ("immunotherapy"). Biological therapies include monoclonal antibodies (including those targeting cancer cell surface, e.g. rituximab and alemtuzumab; anti-CTLA4 Mabs, such as ipilimumab; targeting growth factors, e.g.: bevacizumab, cetuximab, panitumumab and trastuzumab; anti-PD-1 Mabs; anti-Tim3 Mabs; anti-ICOS Mabs), immunoconjugates (e.g.: ⁹⁰Y-ibritumomab tiuxetan, ¹³¹I-tositumomab, and ado-trastuzumab emtansine), cytokines (including interferons such as IFNα; interleukins such as IL-2, IL-11, G-CSM, GM-CSF), therapeutic vaccines (e.g.: Sipuleucel-T (Provenge®)), the bacterium bacillus Calmette-Guérin, cancer-killing viruses, gene therapy, and adoptive T-cell transfer.

### Probiotics

"Probiotics" are micro-organisms that have claimed health benefits when consumed. Probiotics are commonly consumed as part of fermented foods with specially added active live cultures, such as in yogurt, soy yogurt, or as dietary supplements. Generally, probiotics help gut microbiota keep (or re-find) its balance, integrity and diversity. The effects of probiotics are usually strain-dependent.

### Immune checkpoint blockers

In the present text, a "drug blocking an immune checkpoint", or "immune checkpoint blocker" or "immune checkpoint blockade drug" designates any drug, molecule or composition which blocks an immune checkpoint. In particular, it encompasses anti-CTLA-4 antibodies, anti-PD-1 antibodies and anti-PD-L1 antibodies. More particularly, it can be an anti-CTLA-4 monoclonal antibody, especially an anti-CTLA-4 monoclonal IgG1 such as Ipilimumab or an anti-CTLA-4 monoclonal IgG2a such as Tremelimumab.

### Cancer, treatment, etc.

As used herein, "cancer" means all types of cancers. In particular, the cancers can be solid or non solid cancers. Non limitative examples of cancers are carcinomas or adenocarcinomas such as breast, prostate, ovary, lung, pancreas or colon cancer, sarcomas, lymphomas, melanomas, leukemias, germ cell cancers and blastomas.

The immune system plays a dual role against cancer: it prevents tumor cell outgrowth and also sculpts the immunogenicity of the tumor cells. Drugs blocking an immune checkpoint can hence be used to treat virtually any type of cancer. Thus, the methods according to the invention are potentially useful for patients having a cancer selected amongst adrenal cortical cancer, anal cancer, bile duct cancer (e.g. periphilar cancer, distal bile duct cancer, intrahepatic bile duct cancer), bladder cancer, bone cancers (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancers (e.g. meningioma, astocytoma, oligodendrogliomas, ependymoma, gliomas, medulloblastoma, ganglioglioma, Schwannoma, germinoma, craniopharyngioma), breast cancer (e.g. ductal *carcinoma in situ,* infiltrating ductal carcinoma, infiltrating lobular carcinoma, lobular *carcinoma in situ,* gynecomastia), Castleman disease (e.g. giant lymph node hyperplasia, angiofollicular lymph node hyperplasia), cervical cancer, colorectal cancer, endometrial cancers (e.g. endometrial adenocarcinoma, adenocanthoma, papillary serous adnocarcinoma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin's disease, non-Hodgkin's lymphoma, Kaposi's sarcoma, kidney cancer (e.g. renal cell cancer), laryngeal and hypopharyngeal cancer, liver cancers (e.g. hemangioma, hepatic-adenoma, focal nodular hyperplasia, hepatocellular carcinoma), lung cancers (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancers (e.g. seminoma, nonseminoma germ cell cancer), thymus cancer, thyroid cancers (e.g. follicular carcinoma, anaplastic carcinoma, poorly differentiated carcinoma, medullary thyroid carcinoma, thyroid lymphoma), vaginal cancer, vulvar cancer, and uterine cancer (e.g. uterine leiomyosarcoma). More particularly, the method according to the invention can be used for predicting a patient's response to a medicament targeting an immune checkpoint, wherein the patient has a cancer selected from the group consisting of metastatic melanoma. non-small cells lung carcinoma (NSCLC), small cell lung cancer (SCLC), prostate cancer and prostatic neoplasms (especially metastatic hormone-refractory prostate cancer), sarcoma, Wilm's tumor, lymphoma, neuroblastoma, and bladder cancer.

As used herein, the terms "treat", "treatment" and "treating" refer to any reduction or amelioration of the progression, severity, and/or duration of cancer, particularly a solid tumor; for example in a breast cancer, reduction of one or more symptoms thereof that results from the administration of one or more therapies.

Other definitions will be specified below, when necessary.

A first aspect of the present invention is the use of a combination of an immune checkpoint blocker and of an antibiotic selected from the group consisting of vancomycin, penicillin and any antibiotic to which *B.fragilis* strains are resistant, for treating a cancer.

According to a particular embodiment, the antibiotic is vancomycin.

According to another particular embodiment, the immune checkpoint blocker is selected from the group consisting of an anti-CTLA-4 antibody, an anti-PD-1 antibody and an anti-PD-L1 antibody. In particular, an anti-CTLA-4 monoclonal antibody can advantageously be used, such as, for example, an anti-CTLA-4 monoclonal IgG1 (e.g., Ipilimumab) or an anti-CTLA-4 monoclonal IgG2a (e.g., Tremelimumab).

Of course, since the immune system plays an important part against all the cancers, the present invention can benefit to patients who suffer from virtually any cancer. According to a particular aspect, the patient suffers from metastatic melanoma.

As used herein, the term "combination" refers to the use of more than one agent (e.g., vancomycin and Ipilimumab). The use of the term "combination" does not restrict the order in which therapies are administered to the patient, although it is preferable to administer the antibiotic prior to or simultaneously with the immune checkpoint blocker. For example, vancomycin can be administered prior to Ipilimumab (e. g., 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), either punctually or several times (for example, each day) before the antineoplastic treatment is administered. Advantageously, the antibiotic is administered before administration of the drug blocking an immune checkpoint, in order to modulate the patient's gut microbiota to optimize the effect of the immune checkpoint blocker (such as those defined above). The present invention hence provides a method for treating a cancer patient, comprising administering vancomycin and/or penicillin prior to administering a drug blocking an immune checkpoint to said patient.

The present invention also pertains to the use of an antibiotic to which *B. fragilis* strains are resistant, such as, but not limited to, vancomycin and penicillin, as an adjuvant therapy to potentiate the anticancer effects of a drug blocking an immune checkpoint. Indeed, as illustrated in the experimental part below, it can be useful to modulate the gut microbiota of a patient, through the use of antibiotics and/or probiotics, for increasing the anticancer effects of an immune checkpoint blocker. In particular, an antibiotic can be used for increasing the relative amount of Gram negative bacteria, especially the relative amount of *Bacterioides* such as *B. fragilis* in the gut microbiota of a patient before administering an immune checkpoint blocker to said patient (or at the same time). Vancomycin is particularly useful to that aim.

In what precedes, the "relative amount", which can also be designated as the "relative abundance", is defined as the number of bacteria of a particular taxonomic level (from phylum to species) as a percentage of the total number of bacteria in a biological sample. This relative abundance can be assessed, for example, by measuring the percentage of 16S rRNA gene sequences present in the sample which are assigned to these bacteria. It can be measured by any appropriate technique known by the skilled artisan, such as 454 pyrosequencing and quantitative PCR of these specific bacterial 16S rRNA gene markers or quantitative PCR of any gene specific for a bacterial group.

The present invention also relates to the use of a probiotic bacterial composition comprising bacteria selected amongst *Bacteroides fragilis* and *Bacteroides thetaiotaomicron,* in combination with an immune checkpoint blocker as defined above, for inducing immunostimulation in a cancer patient. A method for treating a cancer patient, comprising administering a probiotic bacterial composition comprising bacteria selected from the group consisting of *Bacteroides fragilis, Bacteroides thetaiotaomicron* and mixtures thereof, prior to administering a drug blocking an immune checkpoint to said patient, is hence also part of the present invention.

According to a preferred embodiment, the probiotic bacterial composition is formulated for oral administration. The skilled artisan knows a variety of formulas which can encompass living or killed microorganisms and which can present as food supplements (e.g., pills, tablets and the like) or as functional food such as drinks, fermented yoghurts, *etc.*

This aspect of the present invention is particularly useful for a cancer patient who has a dysbiosis with an under-representation of Gram-negative bacteria and/or who has previously received a broad-spectrum antibiotic treatment.

Another aspect of the present invention is a method for *in vitro* determining whether a cancer patient can benefit from an antineoplastic treatment comprising a drug blocking an immune checkpoint, comprising the following steps:
(i) from an appropriate biological sample from said patient, determining the relative abundance of *Bacteroides fragilis* and *Bacteroides thetaiotaomicron* in said patient's gut microbiota;
(ii) determining the presence or absence of an intestinal dysbiosis;
wherein an intestinal dysbiosis with an under-representation of bacteria recited in step (i) indicates that the patient will not be a good responder to the antineoplastic treatment.

In the present text, a "good responder to a treatment", also called a "responder" or "responsive" patient or in other words a patient who "benefits from" this treatment, refers to a patient who is affected with a cancer and who shows or will show a clinically significant relief in the cancer after receiving this treatment. Conversely, a "bad responder" or "non responder" is one who does not or will not show a clinically significant relief in the cancer after receiving this treatment. The disease clinical data may be assessed according to the standards recognized in the art, such as immune-related response criteria (irRC), WHO or RECIST criteria.

According to a particular embodiment, the biological sample is a biofilm of a biopsy (preferably of a large biopsy) of caecum or colon mucosae obtained from the patient. For example, this biopsy can have been obtained during a specific surgery in pancreatic, stomach, biliary tract or colon cancers.

According to another embodiment, which concerns any type of cancer, the biological sample is a sample of feces obtained from the patient. This sample can have been collected at diagnosis, for example, or at any moment before deciding the beginning of the treatment.

When an intestinal dysbiosis with an under-representation of the "favorable" bacteria listed above is observed, this shows that the patient requires a treatment to balance the gut microbiota prior to starting the antineoplastic treatment with an immune checkpoint blocker, or as an adjuvant of said treatment (e.g.: prebiotics or probiotics administration before/when starting the therapy). Hence, decision can be made to adapt the patient's regimen (providing pre- or probiotics) during a period of time (for example, a few weeks) before beginning the antineoplastic treatment.

According to another aspect, the present invention pertains to a method *for in vitro* determining whether an antineoplastic treatment is to be continued or stopped for a cancer patient, comprising the following steps:
(i) from a biological sample from said patient, obtained 3 to 9 weeks after the beginning of said antineoplastic treatment, analyzing memory CD4⁺ T cell response directed against at least one commensal species of bacteria, for example against *Bacteroides fragilis* and *Bacteroides thetaiotaomicron;*
(ii) for each commensal species against which the CD4⁺ T cell response is analyzed, classifying the response in one of the following categories:
   - no memory CD4⁺ T cell response;
   - memory response of a Th10 phenotype;
   - memory response of a Th1 phenotype,
wherein if a memory response of a Th1 phenotype is observed for at least one commensal species, the antineoplastic treatment is continued, and in absence of such a response, the antineoplastic treatment is stopped.

In order to classify the responses, the secretions of IL-2, TNFα, IFNγ and IL-10 are measured in *ex vivo* restimulation assays. In a preferred embodiment, a first assay is done before the beginning of the treatment, in order to compare the cytokine secretion profile after a few weeks of treatment to that observed pre-treatment. These assays can be performed, for example, using patients' autologous monocytes loaded with defined bacteria and incubated with CD4⁺CD45RO⁺ T cells purified from autologous blood. The response will be classified in the third (favourable) category if it is of a Th1 phenotype, *i.e.,* if restimulation triggers a significant secretion of IL-2, TNFα and IFNγ, and a low secretion of IL-10, especially when comparing the results obtained post- to pre-treatment. Typically, for a patient having a response of the Th1 phenotype, at least a 2-fold increase of IFNγ secretion is observed post-treatment (compared to pre-treatment). The first category (no memory CD4⁺ T cell response) corresponds to the absence of significant cytokine secretion in restimulation assays post-treatment, whereas the second category corresponds to a response in which the IL-10 secretion in a restimulation assay post-treatment is superior to that observed pre-treatment.

One particularly advantageous aspect of this pharmacodynamic method is that it can be performed using a blood sample. Of course, it can be done for patients having any kind of cancers.

To further investigate the role of Gram-negative bacteria in the immune response triggered by anti-CTLA4 molecules, the inventors have tested the response to Ipilimumab in mice lacking a functional TLR4 gene. The toll-like receptor 4, encoded by the TLR4 gene, detects lipopolysaccharide from Gram-negative bacteria and is thus important in the activation of the innate immune system. As shown below, the inventors demonstrated that the antitumor efficacy of CTLA4 blockade is partially dependent on TLR4. Hence, the present invention also pertains to *an in vitro* method of identifying a patient who is unable to fully respond to a drug blocking CTLA4, comprising determining the functionality of the toll-like receptor 4 (TLR 4) in said patient; a patient lacking a functional TLR 4, is identified as not being a good responder to a drug blocking CTLA4. Two cosegregating single nucleotide polymorphisms (SNPs) - Asp299Gly and Thr399Ile - have been identified within the gene encoding TLR4. These SNPs are present in approximately 10% of white individuals, and have been found to be positively correlated with several infectious diseases. In a particular embodiment of this method, the presence or absence of one or both of these SNPs is determined, for example by PCR or by any other method known by the skilled artisan.

Another aspect of the present invention is the use, in adoptive T cell transfer, of a dendritic cell (DC) presenting capsular polysaccharides A (PSA) or PSA and adjuvants (such as TLR3L and anti-CD40 agonistic Ab) or zwitterionic polysaccharide repeated motifs from *B. fragilis* or lysine-aspartic acid (KD) peptides with >15 repetitive units, in combination with an antineoplastic treatment comprising a drug blocking an immune checkpoint, especially in combination with an anti-CTLA4 molecule, for treating cancer. Adoptive T cell transfer specific for *Bacteroides* spp., for example adoptive transfer of T cells amplified or primed *ex vivo* with antigen presenting cells exposed to ZPS or PSA or KD peptides, in a dysbiotic patient bearing a cancer and receiving anti-CTLA4 Ab, is hence an important part of the present invention.

The present invention also relates to a method for *ex vivo* obtaining T cells able to improve the anticancer activity of a drug blocking an immune checkpoint, comprising *ex vivo* expanding a polyclonal T cell line or bulk autologous T cells with dendritic cells (DC) presenting capsular polysaccharides A (PSA) or PSA and adjuvants or zwitterionic polysaccharide (ZPS) repeated motifs from *B. fragilis* or lysine-aspartic acid (KD) peptides with > 15 repetitive units. The cells obtained through this method are advantageously used in adoptive cell transfer of T lymphocytes, in combination with an antineoplastic treatment comprising a drug blocking an immune checkpoint, for treating cancer.

Finally, the present invention also provides anticancer vaccines, comprising PSA and/or ZPS and/or KD. These vaccines are advantageously used in combination with an immune checkpoint blocker.

Other characteristics of the invention will also become apparent in the course of the description which follows of the biological assays which have been performed in the framework of the invention and which provide it with the required experimental support, without limiting its scope.

### EXAMPLES

### EXAMPLE 1 : THE INTESTINAL MICROBIOTA MODULATES THE ANTICANCER IMMUNE EFFECTS OF IPILIMUMAB - MOUSE STUDY

### Materials and Methods

*N.b*.: absent contrary indication, the materials and method which are described in the present example are those which have also been used in the other examples.

**Mice.** All animal experiments were carried out in compliance with French and European laws and regulations. Mice were used between 7 and 14 weeks of age. WT SPF C57BL/6J were obtained from Harlan and kept in specific pathogen-free conditions (SPF). C57BL/6J germ-free mice were obtained from Institut Pasteur and maintained in sterile isolators. *Il-10*-/- C57BL/6J mice were kindly provided by Anne O'Garra (National Institute for Medical Research, UK).

**Cell culture and reagents.** OVA-expressing mouse fibrosarcoma MCA205 cells (class I MHC H-2^{b}, syngenic for C57BL/6 mice) were cultured at 37°C under 5% CO₂ in RPMI-1640 medium supplemented with 10% heat-inactivated fetal bovine serum (FBS), 100 units/ml penicillin G sodium, 100 µg/ml streptomycin sulfate, 2mM L-glutamine, 1mM sodium pyruvate and non-essential amino acids, all from Gibco-Invitrogen (Carlsbad, CA, USA). OVA-expressing MCA205 cells selected in complete medium (as above) further supplemented with 50 µg/ml hygromycin B (Invitrogen, Life Technologies™).

**Tumor challenge and treatment.** Mice were subcutaneously injected in the right flank with 1 x 10⁶ MCA205-OVA. When tumors reached a size of 25 to 40 mm², mice were injected intraperitoneally (i.p) with 100µg of anti-CTLA-4 (clone 9D9) from BioXcell (West Lebanon, NH, USA). Mice were injected 5 times at 3-day intervals with 9D9 and tumor size was routinely monitored by means of a caliper.

**Antibiotics protocols.** Mice were treated with antibiotics 2-3 weeks before tumor implantation and continued until the end of the experiment. A mix of ampicillin (1 mg/ml) + streptomycin (5 mg/ml) + colistin (1 mg/ml) (Sigma-Aldrich) or vancomycine (0.25 mg/ml) or imipenem (0.25 mg/ml) or colistin alone (2.103U/ml) were added in sterile drinking water. Solutions and bottles were changed 2-3 times a week or daily regarding imipenem. Antibiotic activity was analyzed by macroscopic changes observed at the level of caecum (dilatation) and by cultivating the fecal pellets resuspended in BHI+15% glycerol at 0,1g/ml on blood agar plates for 48h at 37°C with 5% CO2 in aerobic or anaerobic conditions.

**Flow cytometry.** Eight-color flow cytometry analysis was performed with antibodies conjugated to fluorescein isothiocyanate, phycoerythrin, phycoerythrin cyanin 7, peridinin chlorophyll protein cyanin 5.5, allophycocyanin cyanin 7, pacific blue, or allophycocyanin. The staining was realized on splenocytes or monocellular suspensions from mouse tumors. Tumors and spleen were harvested two days after the third injection of anti-CTLA-4. Excised tumors were cut into small pieces and digested in RPMI medium containing Liberase™ at 25ng/ml (Roche) and DNase1 at 150UI/ml (Roche) for 30 minutes at 37°C, followed by crushing the pieces on a 100µm cell strainer. Two millions of splenocytes (after red blood cells lysis) or tumor cells were preincubated with purified anti-mouse CD16/CD32 (clone 93 purchased from eBioscience) for 15 minutes at 4°C, before the membrane staining. For intracellular staining, the FoxP3 staining kit (eBioscience) was used. Dead cells were excluded using the Live/Dead Fixable Yellow dead cell stain kit (Life Technologies™). Stained samples were run on a Canto II (BD Bioscience, San Jose, CA, USA) cytometer, and analyses were performed with FlowJo software (Tree Star, Ashland, OR, USA). For cytokine staining, cells were stimulated for 4 hours at 37°C with 50ng/ml of phorbol 12-myristate 13-acetate (PMA), 1µg/ml of ionomycin (Calbiochem) and BD Golgi STOP ™ (BD Biosciences). Anti-CD45.2 (104), FoxP3 (FJK-16s), ICOS (7E17G9), IFN-γ (XMG1.2), TNF-α (MP6-XT22) and isotype controls rat IgG1 (eBRG1), IgG2a (eBRG2a), IgG2b (eBRG2b) were purchased from eBioscience. Anti-CD3 (145-2C11), CD25 (PC61.5.3), KI67 (FITC mouse anti-human KI67 set), rat IgG1κ were obtained from BD Bioscience. Anti-CD4 (GK1.5), CD8β (YTS1567.7), Rat IgG2a (RTK2758) were purchased from Biolegend (San Diego, CA, USA).

**Histology and immunofluorescence of gut tissue.** The whole small intestine (duodenum, jejunum and ileum) and the colon were removed, cleaned from feces and fixed in 4% of PFA for 1h. Rehydratation of the tissue was performed in 15% sucrose for 1h and in 30% sucrose overnight. The small intestine or the colon were entirely rolled, then embedded in optimum cutting temperature (OCT) compound (Sakura), snap frozen and longitudinal or transversal 6 µm sections were prepared. For histological analysis, the longitudinal sections were counterstained with hematoxilin and eosin. For the histological quantitative analysis, inflammatory foci, altered villi and the thickness of lamina propria were scored for each section, while the number of goblet cells was counted for each villus.

**ELISA Lipocalin 2.** After feces collection, individual (not pooled) samples were reconstituted in PBS containing 0.1% Tween 20 (100 mg/ml) and vortexed for 10-20 min to get a homogenous fecal suspension. These samples were then centrifuged for 10 min at 12000 rpm. Clear supernatants were collected and stored at - 20°C until analysis. Lcn-2 levels were estimated in the supernatants using Duoset murine Lcn-2 ELISA kit (R&D Systems, Minneapolis, MN) using manufacturer procedures.

**Bacterial isolation, cultivation and identification.** After five anti-CTLA4 injections, fecal pellets or caecum content were harvested and resuspended in BHI+15% glycerol at 0.1g/ml. Serial dilutions of feces or cecal content from WT or il-*10-*/*-* tumor bearers mice, treated with anti-CTLA4 or Isotype control, treated or not with vancomycine were plated onto blood agar plates and incubated for 48h at 37°C with 5% in aerobic or anaerobic conditions. After 48h, single colonies were isolated and Gram staining was performed. The identification of specific bacteria was accomplished through the combination of morphological tests and the analysis through Andromas (BioMérieux, France) and verified in mass spectrometry (MALDI-TOF).

**Microbiota reconstitution.** For inoculation of GF mice or broad spectrum antibiotics treated mice, colonization was performed the day following the first anti-CTLA4 injection by oral gavage with 100µl of suspension containing 10⁹ bacteria. Efficient colonization was checked by culture of feces 48h post oral gavage. *B. fragilis, B. thetaïotaomicron, B. distasonis and L. plantarum* were grown onto COS agar plates (Biomerieux) for 48h at 37°C with 5% CO2 in anaerobic conditions. *E. Coli* was grown onto COS agar plates for 24h at 37°C with 5% CO2 in aerobic conditions. Bacteria were harvested from the agar plates, then suspended in sterile PBS, centrifuged, washed once and resuspended in sterile PBS at an OD(600nm) of 1, which corresponds approximately to 1x10⁹ colony-forming units (CFU)/ml. Equal volume of each bacteria suspension was mixed to give a suspension of equal proportion of each type of bacteria at 1x10⁹ bacteria/ml.

For experiments with antibiotics treated mice, after 2-3 weeks of antibiotics, the treatment was stopped during the first anti-CTLA4 injection and mice were orally gavaged with 10⁹ CFU the following day.

*P. distasonis* and *E. coli* used in the experiments were isolated from feces of SPF mice treated with anti-CTLA4 and identified as described above. *L. plantarum*, *B. fragilis and B. thetaïotaomicron* were kindly provided by I. Gomperts Boneca, Institut Pasteur, France.

**Isolation of lamina propria cells from colon.** Whole colon was harvested, Peyer's patches were removed, as well as all fat residues and feces. Small fragments were obtained by cutting them first longitudinally along the length and then transversally into pieces of 1-2 cm length. After removing the intra-epithelial lymphocytes (IELs), the gut pieces were further cut and incubated with 0.25 mg/ml collagenase VIII and 10 U/ml DNase I for 40 min at 37 °C under shaking to isolate 5 lamina propria cells (LPCs). After digestion, intestinal pieces were mashed on a cell strainer. For FACS analysis, cell suspensions were subjected to a percoll gradient for 20 min at 2100 RPM. Anti-mouse antibodies for CD45.2 (104), CD3 (145-2C11), CD4 (GK1.5), IFN□ (XMG1.2), ROR□t (AFKJS-9), were obtained from BioLegend, eBioscience and R&D.

**CD4⁺ T cell memory response.** Bone marrow-derived dendritic cells (BMDCs) were generated from femurs and tibiae of C57BL/6 mice, cultured for 7 days in Iscove's medium (Sigma-Aldrich) with J558 supernatant (containing 40 ng/ml of GM-CSF), 10% FCS, 100 IU/ml penicillin/streptomycin, 2 mM L-glutamin, 50 M 2-mercaptoethanol (Sigma-Aldrich) and split every 3-4 days. At day 7, BMDCs were infected with the isolated bacterial strains at a MOI (multiplicity of infection) 1:50 or 1:10 or 1:2 for 1h at 37°C in the appropriate medium without antibiotics. Then, cells were washed with PBS and incubated in complete medium supplemented with gentamicin (50µg/ml) to kill extracellular bacteria. After 24h, BMDCs were cultured together with CD4+ T cells, purified from spleen (Miltenyi) of anti-CTLA4 treated mice at the ratio 1:2 for 24h. Briefly, splenic cells were washed twice and incubated with anti-CD4 magnetic beads at 4°C for 30 min and then separated by magnetic field. When checked by flow cytometry, over 95% of the cells were CD4+ T cells. Co-culture supernatants were then assayed for IL-10 and IFN□ by ELISA.

**Adoptive T cell transfer.** CD4⁺ T cells were isolated from co-culture at a MOI 1:10 as described above, one million of CD4⁺ T cells was adoptively transferred i.v into antibiotics treated mice one day after the first anti-CTLA4 injection. Mice were injected 5 times at 3-day intervals with 9D9 and tumor size was routinely monitored by means of a caliper.

**Statistical analysis.** Data were analyzed with Microsoft Excel (Microsoft Co., Redmont, WA, USA), Prism 5 (GraphPad San Diego, CA, USA). Data are presented as means ± SEM and p-values were computed by paired or unpaired t-tests where applicable. Tumor growth was subjected to a linear mixed effect modeling applied to log pre-processed tumor volumes. The p-values were calculated by testing jointly whether both tumor growth slopes and intercepts (on a log scale) are where dissimilar between treatment groups of interests. All reported tests are two-tailed and were considered significant for p-values <0.05.

### Results

Ipilimumab induced immune-mediated long-term control of metastatic melanoma (MM) in a fraction of the patients at the Gustave Roussy Cancer Center (Delyon et al, 2013; Menard et al, 2008), calling for biomarker discovery. The key role of the CD25/CD122 IL-2 receptor complex in the mechanism of action of ipilimumab was unraveled. CTLA-4 blockade led to the elimination of a suppressive CD4⁺ T cell subset expressing Lag3, ICOS, IL-10 and Egr2 with a concomitant raise in IL-2-producing effector cells that lost Foxp3 expression and accumulated in regressing tumors. When combined with CTLA-4 blockade, recombinant IL-2 improved, while its decoy IL-2Ra (sCD25) blocked, anticancer effects by expanding Tregs. In 262 MM patients receiving Ipilimumab, baseline serum concentrations of sCD25 represented an independent indicator of overall survival, with high levels predicting resistance to therapy. Hence, our results revealed a role for IL-2 and IL-2 receptors in the anticancer activity of CTLA-4 blockade (Hannani, Vétizou, Cell Res in press).

With the final aim to uncouple efficacy and toxicity of Ipilimumab in patients, the inventors analyzed the changes and role of gut microbiota during CTLA4 blockade in preclinical models. The results indicate that broad-spectrum antibiotics or monotherapies with imipenem or colistine or metronidazole severely compromise tumor growth reduction by CTLA4 blockade, while vancomycine ameliorates it (Figure 1a, c). The deleterious effects of antibiotics on the anticancer effects of anti-CTLA4 Ab have been observed in three transplantable tumor models (MCA205 sarcomas and MC38 colon cancers, both growing on C57BL/6 mice, as well as CT26 colon cancers growing on BALB/c mice). A loss of efficacy of CTLA4 blockade was also observed in germ-free (GF) mice (Figure 1b).

Antibiotic-treated or GF mice both exhibited reduced intratumoral accumulation of CD45⁺ cells (including neutrophils, CD3⁺ T lymphocytes, as well as polyfunctional CD4⁺ and CD8⁺ T cells), and dampened proliferation of splenic Foxp3⁻ T cells and ICOS expression on CD4⁺ T splenocytes, hallmarks of CTLA4 blockade (Figure 2a). In contrast, the profound ADCC-dependent depletion of intratumoral Treg mediated by the IgG2a anti-CTLA4 Ab was not influenced by the gut microbiota (Figure 2b).

In the gut, anti-CTLA4 mAb disrupted the integrity of the epithelial barrier, increased goblet cell numbers in the small intestine, promoted the release of the antimicrobial peptide lipocalin-2 into the caecum and feces and upregulated RORgt and IFNg in colonic lamina propria (LP) T cells (Figure 3).

Colon toxicity induced by CTLA4 blockade was absent in GF mice, yet was exacerbated in antibiotic-treated mice. In conclusion, Gram-negative intestinal bacteria were mandatory for the immunostimulatory effects of CTLA4 blockade associated with anticancer effects, and gut toxicity was induced by dysbiosis. In *Tlr4*^{*-*/-}mice, the antitumor effects of CTLA4 blockade were only partially reduced.

Pyrosequencing analyses of 16S rRNA gene amplicons from feces and plating stool on agar selective for different species (including *Bacteroides* under anaerobic conditions) revealed a dramatic enrichment in *Bacteroidales* and more specifically of *B.distasonis* and *B. uniformis,* and to a lesser extent of *E. Coli,* to the expense of *Clostridiales,* right after one single injection of anti-CTLA4 mAb (Figure 5a, b).

Importantly, mono-association by oral gavage of tumor bearing-ATB-treated or GF mice with *B. fragilis* or *B. thetaiotaomicron* restored the anticancer effects of CTLA4 blockade (while *B.distasonis, E. Coli, Lactobacilli* failed to do so) (Figure 6).

Interestingly, the colitogenic effects of CTLA4 blockade in ATB-tumor bearers were partially restored by oral gavage with *B. fragilis* but not *E. Coli.* Mono-colonization of tumor bearing- GF mice with *B. fragilis* revealed increased Ki67 and ICOS expression by Foxp3⁺ CD25^{high} Treg as well as decreased proportions of CCR6⁺ CD4⁺ Th17 cells in the secondary lymphoid organs (not shown, (Round et al, 2011). CTLA4 blockade slightly reduced these tolerogenic effects *of B. fragilis* (not shown). Co-blockade of CTLA4 & ICOS or of CTLA4 & IL-10 enhanced the systemic pTH17 responses, exacerbated the colitis and compromised the antitumor effects of anti-CTLA4 Ab, suggesting that the anti-CTLA4 mAb does not simply act by suppressing tolerance (not shown).

Instead, mice administered with anti-CTLA4 Ab developed memory CD4⁺ TH1 immune responses with drastically reduced IL-10 release, against *B. fragilis* or *B. thetaiotaomicron* but not *B. distasonis* nor *B. uniformis* (Figure 5c). Hence, anti-CTLA4 Ab may uncover unexpected immunostimulatory pathways. Supporting this view, the inventors demonstrated that BM-DC pulsed with *B. fragilis* produce IL-12p40 and that anti-IL-12p70 neutralizing antibodies curtailed the efficacy of anti-CTLA4 Ab in WT mice (not shown).

### EXAMPLE 2 : ADOPTIVE T CELL TRANSFER PRIMED WITH B. FRAGILIS AMELIORATES THE ANTITUMOR EFFECTS OF CTLA4 BLOCKADE IN ABX-TREATED MICE

The protocol used for this experiment is shown in Fig. 7a and 7b.

*Ex vivo* propagated splenic CD4⁺ T cells stimulated in the presence of BM-DC loaded with *Bacteroides spp.* such as *B. fragilis* were adoptively transferred intravenously into ATB-treated MCA205-OVA tumor bearers. As shown in figure 7c, CD4+ T cells primed with *Bacteroides fragilis* could rescue the negative impact of broad spectrum antibiotics on the antitumor efficacy of the anti-CTLA4. Such effects were not observed with CD4⁺ T cells primed with BM-DC without bacteria.

### REFERENCES

Baeck C, Wei X, Bartneck M, Fech V, Heymann F, Gassler N, Hittatiya K, Eulberg D, Luedde T, Trautwein C, Tacke F (2014) Pharmacological inhibition of the chemokine C-C motif chemokine ligand 2 (monocyte chemoattractant protein 1) accelerates liver fibrosis regression by suppressing Ly-6C(+) macrophage infiltration in mice. Hepatology 59: 1060-1072
Beck KE, Blansfield JA, Tran KQ, Feldman AL, Hughes MS, Royal RE, Kammula US, Topalian SL, Sherry RM, Kleiner D, Quezado M, Lowy I, Yellin M, Rosenberg SA, Yang JC (2006) Enterocolitis in patients with cancer after antibody blockade of cytotoxic T-lymphocyte-associated antigen 4. Journal of clinical oncology : official journal of the American Society of Clinical Oncology 24: 2283-2289
Berman D, Parker SM, Siegel J, Chasalow SD, Weber J, Galbraith S, Targan SR, Wang HL (2010) Blockade of cytotoxic T-lymphocyte antigen-4 by ipilimumab results in dysregulation of gastrointestinal immunity in patients with advanced melanoma. Cancer immunity 10: 11
Blaser M (2011) Antibiotic overuse: Stop the killing of beneficial bacteria. Nature 476: 393-394
Cabrera S, Fernandez AF, Marino G, Aguirre A, Suarez MF, Espanol Y, Vega JA, Laura R, Fueyo A, Fernandez-Garcia MS, Freije JM, Kroemer G, Lopez-Otin C (2013) ATG4B/autophagin-1 regulates intestinal homeostasis and protects mice from experimental colitis. Autophagy 9: 1188-1200
Dasgupta S, Erturk-Hasdemir D, Ochoa-Reparaz J, Reinecker HC, Kasper DL (2014) Plasmacytoid dendritic cells mediate anti-inflammatory responses to a gut commensal molecule via both innate and adaptive mechanisms. Cell host & microbe 15: 413-423
Delyon J, Mateus C, Lefeuvre D, Lanoy E, Zitvogel L, Chaput N, Roy S, Eggermont AM, Routier E, Robert C (2013) Experience in daily practice with ipilimumab for the treatment of patients with metastatic melanoma: an early increase in lymphocyte and eosinophil counts is associated with improved survival. Annals of oncology : official journal of the European Society for Medical Oncology / ESMO 24: 1697-1703
Faith JJ, Ahern PP, Ridaura VK, Cheng J, Gordon JI (2014) Identifying gut microbe-host phenotype relationships using combinatorial communities in gnotobiotic mice. Science translational medicine 6: 220ra211
Ginhoux F, Jung S (2014) Monocytes and macrophages: developmental pathways and tissue homeostasis. Nature reviews Immunology 14: 392-404
Hall AO, Beiting DP, Tato C, John B, Oldenhove G, Lombana CG, Pritchard GH, Silver JS, Bouladoux N, Stumhofer JS, Harris TH, Grainger J, Wojno ED, Wagage S, Roos DS, Scott P, Turka LA, Cherry S, Reiner SL, Cua D, Belkaid Y, Elloso MM, Hunter CA (2012) The cytokines interleukin 27 and interferon-gamma promote distinct Treg cell populations required to limit infectian-induced pathology. Immunity 37: 511-523
Hamid O, Robert C, Daud A, Hodi FS, Hwu WJ, Kefford R, Wolchok JD, Hersey P, Joseph RW, Weber JS, Dronca R, Gangadhar TC, Patnaik A, Zarour H, Joshua AM, Gergich K, Elassaiss-Schaap J, Algazi A, Mateus C, Boasberg P, Tumeh PC, Chmielowski B, Ebbinghaus SW, Li XN, Kang SP, Ribas A (2013) Safety and tumor responses with lambrolizumab (anti-PD-1) in melanoma. The New England journal of medicine 369: 134-144
Hepworth MR, Monticelli LA, Fung TC, Ziegler CG, Grunberg S, Sinha R, Mantegazza AR, Ma HL, Crawford A, Angelosanto JM, Wherry EJ, Koni PA, Bushman FD, Elson CO, Eberl G, Artis D, Sonnenberg GF (2013) Innate lymphoid cells regulate CD4+ T-cell responses to intestinal commensal bacteria. Nature 498: 113-117
Hodi FS, O'Day SJ, McDermott DF, Weber RW, Sosman JA, Haanen JB, Gonzalez R, Robert C, Schadendorf D, Hassel JC, Akerley W, van den Eertwegh AJ, Lutzky J, Lorigan P, Vaubel JM, Linette GP, Hogg D, Ottensmeier CH, Lebbe C, Peschel C, Quirt I, Clark JI, Wolchok JD, Weber JS, Tian J, Yellin MJ, Nichol GM, Hoos A, Urba WJ (2010) Improved survival with ipilimumab in patients with metastatic melanoma. The New England journal of medicine 363: 711-723
Huang JY, Lee SM, Mazmanian SK (2011) The human commensal Bacteroides fragilis binds intestinal mucin. Anaerobe 17: 137-141
Iida N, Dzutsev A, Stewart CA, Smith L, Bouladoux N, Weingarten RA, Molina DA, Salcedo R, Back T, Cramer S, Dai RM, Kiu H, Cardone M, Naik S, Patri AK, Wang E, Marincola FM, Frank KM, Belkaid Y, Trinchieri G, Goldszmid RS (2013) Commensal bacteria control cancer response to therapy by modulating the tumor microenvironment. Science 342: 967-970
Iwama S, De Remigis A, Callahan MK, Slovin SF, Wolchok JD, Caturegli P (2014) Pituitary expression of CTLA-4 mediates hypophysitis secondary to administration of CTLA-4 blocking antibody. Science translational medicine 6: 230ra245
Koropatkin N, Martens EC, Gordon JI, Smith TJ (2009) Structure of a SusD homologue, BT1043, involved in mucin O-glycan utilization in a prominent human gut symbiont. Biochemistry 48: 1532-1542
Lam W, Bussom S, Guan F, Jiang Z, Zhang W, Gullen EA, Liu SH, Cheng YC (2010) The four-herb Chinese medicine PHY906 reduces chemotherapy-induced gastrointestinal toxicity. Science translational medicine 2: 45ra59
Lee SM, Donaldson GP, Mikulski Z, Boyajian S, Ley K, Mazmanian SK (2013) Bacterial colonization factors control specificity and stability of the gut microbiota. Nature 501: 426-429
Li J, Jia H, Cai X, Zhong H, Feng Q, Sunagawa S, Arumugam M, Kultima JR, Prifti E, Nielsen T, Juncker AS, Manichanh C, Chen B, Zhang W, Levenez F, Wang J, Xu X, Xiao L, Liang S, Zhang D, Zhang Z, Chen W, Zhao H, Al-Aama JY, Edris S, Yang H, Wang J, Hansen T, Nielsen HB, Brunak S, Kristiansen K, Guarner F, Pedersen O, Dore J, Ehrlich SD, Meta HITC, Bork P, Wang J, Meta HITC (2014) An integrated catalog of reference genes in the human gut microbiome. Nature biotechnology 32: 834-841
Ma Y, Yamazaki T, Yang H, Kepp O, Galluzzi L, Zitvogel L, Smyth MJ, Kroemer G (2013) Tumor necrosis factor is dispensable for the success. of immunogenic anticancer chemotherapy. Oncoimmunology 2: e24786
Martens EC, Chiang HC, Gordon JI (2008) Mucosal glycan foraging enhances fitness and transmission of a saccharolytic human gut bacterial symbiont. Cell host & microbe 4: 447-457
Mazmanian SK, Round JL, Kasper DL (2008) A microbial symbiosis factor prevents intestinal inflammatory disease. Nature 453: 620-625
Menard C, Ghiringhelli F, Roux S, Chaput N, Mateus C, Grohmann U, Caillat-Zucman S, Zitvogel L, Robert C (2008) Ctla-4 blockade confers lymphocyte resistance to regulatory T-cells in advanced melanoma: surrogate marker of efficacy of tremelimumab? Clinical cancer research an official journal of the American Association for Cancer Research 14: 5242-5249
Mortha A, Chudnovskiy A, Hashimoto D, Bogunovic M, Spencer SP, Belkaid Y, Merad M (2014) Microbiota-dependent crosstalk between macrophages and ILC3 promotes intestinal homeostasis. Science 343: 1249288
Nielsen HB, Almeida M, Juncker AS, Rasmussen S, Li J, Sunagawa S, Plichta DR, Gautier L, Pedersen AG, Le Chatelier E, Pelletier E, Bonde I, Nielsen T, Manichanh C, Arumugam M, Batto JM, Quintanilha Dos Santos MB, Blom N, Borruel N, Burgdorf KS, Boumezbeur F, Casellas F, Dore J, Dworzynski P, Guarner F, Hansen T, Hildebrand F, Kaas RS, Kennedy S, Kristiansen K, Kultima JR, Leonard P, Levenez F, Lund O, Moumen B, Le Paslier D, Pons N, Pedersen O, Prifti E, Qin J, Raes J, Sorensen S, Tap J, Tims S, Ussery DW, Yamada T, Meta HITC, Renault P, Sicheritz-Ponten T, Bork P, Wang J, Brunak S, Ehrlich SD, Meta HITC (2014) Identification and assembly of genomes and genetic elements in complex metagenomic samples without using reference genomes. Nature biotechnology 32: 822-828
Page DB, Postow MA, Callahan MK, Wolchok JD (2013) Checkpoint modulation in melanoma: an update on ipilimumab and future directions. Current oncology reports 15: 500-508
Patel KK, Stappenbeck TS (2013) Autophagy and intestinal homeostasis. Annual review of physiology 75: 241-262
Peggs KS, Quezada SA, Korman AJ, Allison JP (2006) Principles and use of anti-CTLA4 antibody in human cancer immunotherapy. Current opinion in immunology 18: 206-213
Robert C, Thomas L, Bondarenko I, O'Day S, M DJ, Garbe C, Lebbe C, Baurain JF, Testori A, Grob JJ, Davidson N, Richards J, Maio M, Hauschild A, Miller WH, Jr., Gascon P, Lotem M, Harmankaya K, Ibrahim R, Francis S, Chen TT, Humphrey R, Hoos A, Wolchok JD (2011) Ipilimumab plus dacarbazine for previously untreated metastatic melanoma. The New England journal of medicine 364: 2517-2526
Rossini A, Rumio C, Sfondrini L, Tagliabue E, Morelli D, Miceli R, Mariani L, Palazzo M, Menard S, Balsari A (2006) Influence of antibiotic treatment on breast carcinoma development in proto-neu transgenic mice. Cancer research 66: 6219-6224
Round JL, Lee SM, Li J, Tran G, Jabri B, Chatila TA, Mazmanian SK (2011) The Toll-like receptor 2 pathway establishes colonization by a commensal of the human microbiota. Science 332: 974-977
Sandborn WJ, Feagan BG, Rutgeerts P, Hanauer S, Colombel JF, Sands BE, Lukas M, Fedorak RN, Lee S, Bressler B, Fox I, Rosario M, Sankoh S, Xu J, Stephens K, Milch C, Parikh A, Group GS (2013) Vedolizumab as induction and maintenance therapy for Crohn's disease. The New England journal of medicine 369: 711-721
Sandoval F, Terme M, Nizard M, Badoual C, Bureau MF, Freyburger L, Clement O, Marcheteau E, Gey A, Fraisse G, Bouguin C, Merillon N, Dransart E, Tran T, Quintin-Colonna F, Autret G, Thiebaud M, Suleman M, Riffault S, Wu TC, Launay O, Danel C, Taieb J, Richardson J, Zitvogel L, Fridman WH, Johannes L, Tartour E (2013) Mucosal imprinting of vaccine-induced Cod8(+) T cells is crucial to inhibit the growth of mucosal tumors. Science translational medicine 5: 172ra120
Stingele F, Corthesy B, Kusy N, Porcelli SA, Kasper DL, Tzianabos AO (2004) Zwitterionic polysaccharides stimulate T cells with no preferential V beta usage and promote anergy, resulting in protection against experimental abscess formation. Journal of immunology 172: 1483-1490
Stringer AM, Gibson RJ, Bowen JM, Keefe DM (2009) Chemotherapy-induced modifications to gastrointestinal microflora: evidence and implications of change. Current drug metabolism 10: 79-83
Surana NK, Kasper DL (2012) The yin yang of bacterial polysaccharides: lessons learned from B. fragilis PSA. Immunol Rev 245: 13-26
Tivol EA, Borriello F, Schweitzer AN, Lynch WP, Bluestone JA, Sharpe AH (1995) Loss of CTLA-4 leads to massive lymphoproliferation and fatal multiorgan tissue destruction, revealing a critical negative regulatory role of CTLA-4. Immunity 3: 541-547
Tzianabos AO, Chandraker A, Kalka-Moll W, Stingele F, Dong VM, Finberg RW, Peach R, Sayegh MH (2000a) Bacterial pathogens induce abscess formation by CD4(+) T-cell activation via the CD28-B7-2 costimulatory pathway. Infection and immunity 68: 6650-6655
Tzianabos AO, Finberg RW, Wang Y, Chan M, Onderdonk AB, Jennings HJ, Kasper DL (2000b) T cells activated by zwitterionic molecules prevent abscesses induced by pathogenic bacteria. The Journal of biological chemistry 275: 6733-6740
Tzianabos AO, Onderdonk AB, Rosner B, Cisneros RL, Kasper DL (1993) Structural features of polysaccharides that induce intra-abdominal abscesses. Science 262: 416-419
van Vliet MJ, Harmsen HJ, de Bont ES, Tissing WJ (2010) The role of intestinal microbiota in the development and severity of chemotherapy-induced mucositis. PLoSpathogens 6: e1000879
Velicer CM, Heckbert SR, Lampe JW, Potter JD, Robertson CA, Taplin SH (2004) Antibiotic use in relation to the risk of breast cancer. JAMA : the journal of the American Medical Association 291: 827-835
Viaud S, Daillere R, Boneca IG, Lepage P, Pittet MJ, Ghiringhelli F, Trinchieri G, Goldszmid R, Zitvogel L (2014a) Harnessing the intestinal microbiome for optimal therapeutic immunomodulation. Cancer research 74: 4217-4221
Viaud S, Daillere R, Yamazaki T, Lepage P, Boneca I, Goldszmid R, Trinchieri G, Zitvogel L (2014b) Why should we need the gut microbiota to respond to cancer therapies? Oncoimmunology 3: e27574
Viaud S, Saccheri F, Mignot G, Yamazaki T, Daillere R, Hannani D, Enot DP, Pfirschke C, Engblom C, Pittet MJ, Schlitzer A, Ginhoux F, Apetoh L, Chachaty E, Woerther PL, Eberl G, Berard M, Ecobichon C, Clermont D, Bizet C, Gaboriau-Routhiau V, Cerf-Bensussan N, Opolon P, Yessaad N, Vivier E, Ryffel B, Elson CO, Dore J, Kroemer G, Lepage P, Boneca IG, Ghiringhelli F, Zitvogel L (2013) The intestinal microbiota modulates the anticancer immune effects of cyclophosphamide. Science 342: 971-976
Vingert B, Adotevi O, Patin D, Jung S, Shrikant P, Freyburger L, Eppolito C, Sapoznikov A, Amessou M, Quintin-Colonna F, Fridman WH, Johannes L, Tartour E (2006) The Shiga toxin B-subunit targets antigen in vivo to dendritic cells and elicits anti-tumor immunity. European journal of immunology 36: 1124-1135
Walters MJ, Wang Y, Lai N, Baumgart T, Zhao BN, Dairaghi DJ, Bekker P, Ertl LS, Penfold ME, Jaen JC, Keshav S, Wendt E, Pennell A, Ungashe S, Wei Z, Wright JJ, Schall TJ (2010) Characterization of CCX282-B, an orally bioavailable antagonist of the CCR9 chemokine receptor, for treatment of inflammatory bowel disease. The Journal ofpharmacology and experimental therapeutics 335: 61-69
Weber J, Thompson JA, Hamid O, Minor D, Amin A, Ron I, Ridolfi R, Assi H, Maraveyas A, Berman D, Siegel J, O'Day SJ (2009) A randomized, double-blind, placebo-controlled, phase II study comparing the tolerability and efficacy of ipilimumab administered with or without prophylactic budesonide in patients with unresectable stage III or IV melanoma. Clinical cancer research : an official journal of the American Association for Cancer Research 15: 5591-5598
Xia S, Wei J, Wang J, Sun H, Zheng W, Li Y, Sun Y, Zhao H, Zhang S, Wen T, Zhou X, Gao JX, Wang P, Wu Z, Zhao L, Yin Z (2014) A requirement of dendritic cell-derived interleukin-27 for the tumor infiltration of regulatory T cells. Journal of leukocyte biology

## Claims

1. A combination of a drug blocking an immune checkpoint and of an antibiotic selected from the group consisting of vancomycin and penicillin, for use in the treatment of cancer.

2. The combination of claim 1, for the use according to claim 1, wherein the antibiotic is vancomycin.

3. The combination of claim 1 or claim 2, for the use according to claim 1, wherein said drug blocking an immune checkpoint is an anti-CTLA-4 monoclonal antibody.

4. The combination of any of claims 1 to 3, for the use according to claim 1, wherein the antibiotic is administered to a patient suffering from a cancer before administration of the drug blocking an immune checkpoint to said patient.

5. An antibiotic selected from the group consisting of vancomycin and penicillin, for use for modulating the gut microbiota of a patient to potentiate the anticancer effects of a drug blocking an immune checkpoint administered to said patient.

6. The antibiotic of claim 5, for the use according to claim 5, wherein said drug blocking an immune checkpoint is an anti-CTLA-4 monoclonal antibody.

7. *An in vitro* method of identifying a patient who cannot be a good responder to a drug blocking CTLA4, comprising determining the functionality of the toll-like receptor 4 (TLR 4) in said patient, wherein if said patient lacks a functional TLR 4, the patient is identified as not being a good responder to a drug blocking CTLA4.

8. A probiotic bacterial composition comprising bacteria selected from the group consisting of *Bacteroides fragilis*, *Bacteroides thetaiotaomicron* and mixtures thereof, for use in combination with a drug blocking an immune checkpoint for inducing immunostimulation in a cancer patient.

9. The probiotic bacterial composition according to claim 8, for the use according to claim 8, wherein said composition is formulated for oral administration.

10. The probiotic bacterial composition according to claims 8 or claim 9, for use in combination with an anti-CTLA4 monoclonal antibody, for treating a cancer patient who has a dysbiosis with an under-representation of Gram-negative bacteria.

11. A dendritic cell (DC) presenting capsular polysaccharides A (PSA) or PSA and adjuvants or zwitterionic polysaccharide (ZPS) repeated motifs from *B. fragilis* or lysine-aspartic acid (KD) peptides with >15 repetitive units, for use in adoptive cell transfer (DC), in combination with an antineoplastic treatment comprising a drug blocking an immune checkpoint, for treating cancer.

12. The dendritic cell according to claim 11, for the use according to claim 11, wherein said drug blocking an immune checkpoint is an anti-CTLA 4 molecule.

13. An anticancer vaccine composition, **characterized in that** it comprises PSA and/or KD and/or ZPS.

14. The anticancer vaccine composition according to claim 13, for use in combination with a drug blocking an immune checkpoint for inducing immunostimulation in a cancer patient.

15. A polyclonal T cell population or bulk autologous T cells *ex vivo* expanded with dendritic cell (DC) presenting capsular polysaccharides A (PSA) or PSA and adjuvants or zwitterionic polysaccharide (ZPS) repeated motifs from *B. fragilis* or lysine-aspartic acid (KD) peptides with >15 repetitive units, for use in adoptive cell transfer of T lymphocytes, in combination with an antineoplastic treatment comprising a drug blocking an immune checkpoint, for treating cancer.
